# EUROPÄISCHE PATENTSCHRIFT

(11) **EP 2 576 643 B1**
(45) Veröffentlichungstag und Bekanntmachung des Hinweises auf die Patenterteilung: **04.03.2015**
(21) Anmeldenummer: 11708890.6
(22) Anmeldetag: 21.03.2011
(51) Int. Cl.: C08F 220/18, C08F 220/26, C08F 2/22, C08F 220/04, A61K 8/81, A61Q 5/00, A61Q 19/10

(54) **VERDICKTES POLYMER**
THICKENING POLYMER
POLYMÈRE ÉPAISSISANT

(30) Priorität: 31.05.2010 DE 102010022063
(43) Veröffentlichungstag der Anmeldung: 10.04.2013
(73) Patentinhaber: Beiersdorf AG, 20253 Hamburg (DE)
(72) Erfinder: ALEKSANDROVIC-BONDZIC, Vesna, 22307 Hamburg (DE); MERTENS, Sascha, 21465 Reinbek (DE); FÖRSTER, Stephan, 95448 Bayreuth (DE)
(86) Internationale Anmeldenummer: PCT/EP2011/054247
(87) Internationale Veröffentlichungsnummer: WO 2011/151091

(56) Entgegenhaltungen:
- WO-A1-03/061615
- WO-A1-2005/030163

## Beschreibung

Die Erfindung betrifft ein neuartiges verdickendes Polymer mit besonderen Eigenschaften, das frei von assoziativen Monomeren ist bzw dessen Kette keine solchen assoziativen Monomere enthält.

Alle Mengenangaben sind- sofern nicht anders angegeben - Massenprozente bezogen auf die Gesamtmasse der Zubereitung.

Assoziative Monomere im Sinne der vorliegenden Schrift sind Monomere vom Typ U-S-L , wobei U eine ethylenisch ungesättigte Gruppe wie Methacrylat-, Acrylat-, Allyl- oder Vinylgruppen enthält, S ein Spacer aus einer Polyoxyalkylenkette mit 2 bis 300 Alkylenoxyeinheiten wie Polyethylenglykol, Polypropylenglykol oder Polybutylenglykol ist und L eine Hydrophobe Gruppe wie eine Alkyl- und/oder Arylgruppe ist, die wenigstens 4, bevorzugt wenigstens 8 Kohlenstoffatome aufweist, beispielsweise eine Laurylgruppe.

tan δ -Wert im Sinne der vorliegenden Schrift bedeutet der Quotient aus dem Verlustmodul und dem Speichermodul, jeweils gemessen bei 40°C. Dabei kommt es auf das verwendete Messgerät nicht an. Die in dieser Schrift angegebenen Werte wurden mit dem Malvern Gemini HR nano bestimmt.

Allgemein dienen verdickende Polymere dazu, Wasser oder wasserhaltige Zubereitungen zu verdicken. Dabei lässt das Wasser die Polymere zu Gelen aufquellen um so Viskosität und Fließverhalten zu beeinflussen. Verdickende Polymere sind von Bedeutung bei der Herstellung von Körperreinigungsmitteln, Cremes, Reinigungsmitteln, Appreturen, Druckfarben, Lacken, Dispersionsfarben und anderen Beschichtungsstoffen, Klebstoffen, Papier, Lebensmitteln und so fort.

Aus der EP 1465932 B1 waren Alkali-quellbare und alkalilösliche assoziative Mehrzweck-Polymere, die das Polymerisationsprodukt eines Monomergemisches aus
a) mindestens einem säurehai'tigen Vinyl-Monomer,
b) mindestens einem nichtionischem Vinyl-Monomer,
c) einem ersten assoziativen Monomer, das eine erste hydrophobe Endgruppe hat,
d) einem zweiten assoziativen Monomer mit einer zweiten hydrophoben Endgruppe, einem halbhydrophoben Monomer und aus einer Kombination davon und optional
e) einem oder mehreren querverbindenen Monomeren oder Kettenüberträgern ist, bekannt.

Solche Polymere werden durch Emulsionspolymerisation hergestellt. Dabei wird zunächst die Monomermischung mit einem Tensid in Wasser In Form von Mizellen emulgiert oder suspendiert. In den eigentlichen Reaktor wird der Initiator in wässriger Lösung oder Suspension vorgelegt und die Monomersuspension langsam zugegeben. Die Geschwindigkeit der Monomerzugabe wird so gesteuert, dass es nicht zu einem erheblichen Anstieg der Reaktionstemperatur durch den Trommsdorff-Effekt (Geleffekt) kommt. Der Geleffekt tritt bei einem zu starken Anstieg des Umsatzes auf und führt dazu, dass die Polymerisationsgeschwindigkeit durch Hinderung der Diffusion der Polymorradikate ansteigt und sich die Polymerisation selbst beschleunigt. Dies versucht man aus Gründen der Sicherheit und zur Erhalt einer engen Molmassenverteilung zu verhindern. Tritt der Geleffekt auf, so erhält man eine bimodale Molmassenverteilung.

Weiterer Stand der Technik findet sich in EP 1272159, WO0306228 WO03061615, WO03062288, WO2007090759. US6242531.

Wünschenswert sind verdickende Polymere, mit denen es möglich Ist, wässrige Zubereitungen so zu verdicken, dass möglichst klare Gele entstehen.

Weiterhin wünschenswert sind verdickende Polymere, mit denen in wässriger Lösung möglichst geringe tan δ-Werte erreicht werden können.

Ebenfalls wünschenswert sind verdickende Polymere mit einer deutlich erweiterten Lagerstabilität.

Dem Stand der Technik fehlte es an verdickenden Polymeren, die die Herstellung klarer Gele bei gleichzeitig geringen tan δ-Werten ermöglichen.

Überraschend und für den Fachmann nicht vorhersehbar, hat sich nun herausgestellt, dass ein Polymer erhältlich durch radikalische Emulsionspolymerisation von
(A) wenigstens einem sauren Vinylmonomer oder dessen Salz,
(B) wenigstens einem nichtionischen Vinylmonomer, besonders bevorzugt einem hydrophoben nichtionischen Vinylmonomer,
(C) wenigstens einem Monomer, enthaltend eine ungesättigte Endgruppe und einen Polyoxyalkylenteil,
(D) wenigstens einem quervemetzenden Monomeren,
(E) optional einem Schutzkolloid dadurch gekennzeichnet, dass die Polymerisation so gesteuert wird, dass zumindest zeitweise der Geleffekt auftritt, dadurch erreicht, dass die Monomerzugabe (Dosierzeit),während 40 Minuten, besonders bevorzugt während 30 Minuten erfolgt und dadurch gekennzeichnet, dass (F) assoziative Monomere fehlen, den Mängeln des Standes der Technik abhilft. Bei einer solchen Polymerisation unter Ausnutzung des Trommsdorff-Effektes, d.h. bei konstanter Zugabe der Monomere und gleichzeitig hoher Zugabegeschwindigkeit der Monomere bildet sich ein Monomerenüberschuss, der zu einer Selbstbeschleunigung der Polymerisation (Trommsdorff-Effekt) führt. Das Ergebnis Ist ein Anstieg der Molekulargewichte bei gleichzeitig vorteilhafter Morphologie der Polymere. Besonders bevorzugt ist es, wenn das saure Vinylmonomer (A) gewählt wird unter Vinylmonomeren mit Carboxylgruppen, besonders bevorzugt Acrylsäure oder Methacrylsäure oder deren Alkali-, Erdalkali-, Ammonium oder Alkylammoniumsalzen, ganz besonders bevorzugt Methacrylsäure oder deren Alkali-, Erdalkali-, Ammonium oder Alkylammoniumsalze. Besonders bevorzugt ist es, wenn das nichtionische Vinylmonomer (B) gewählt wird unter C1-C22-Alkyl-(meth)acrylaten und deren Mischungen. Dadurch werden gute Fließeigenschaften und damit ein vorteilhaftes rheologisches Profil erreicht. Zusätzliche langkettige Alkylacrylate (C12 und C18) sind besonders bevorzugt, weil sie die erreichbare Viskosität erhöhen. Besonders bevorzugt ist es, wenn das Monomer (C), enthaltend eine ungesättigte Endgruppe und einen Polyoxyalkylenteil gewählt wird unter Vinylpolyalkylenglykolen oder polymerisierbaren Tensiden oder deren Mischungen, besonders bevorzugt gewählt wird unter R307, RAL307, A11/1800, R1100, A111 R, AB25-8. Besonders bevorzugt ist es, wenn das quervernetzende Monomer (D) gewählt wird unter Polyol(meth)acrylaten mit wenigstens zwei (Meth)acrylatgruppen und den Mischestern von Polyolen mit Acrylsäure und/oder Methacrylsäure. Weiter besonders bevorzugt ist es, wenn die Monomere (A) in Gehalten von 10 bis 75 %, bevorzugt 30 bis 50 %, besonders bevorzugt 35 bis 49 %, (B) in Gehalten von 10 bis 90 %, bevorzugt 30 bis 80 %, besonders bevorzugt 47 bis 60 %, (C) in Gehalten von 0,5 bis 40 %, bevorzugt 1 bis 10 %, besonders bevorzugt 2 bis 6 %, (D) in Gehalten von bis zu 1 %, bevorzugt 0,05 bis 0,5 %, besonders bevorzugt 0,1 bis 0,35 % vorliegen .Ganz besonders bevorzugt ist es, wenn die Monomere (A):(B) in Massenverhältnissen von 1:2 bis 2:1 vorliegen. Ganz besonders bevorzugt ist es, wenn das Polymer unter Beispiel 22. 24, 25, 28, 35, 37, oder 40 gewählt wird. De Erfindung umfasst auch eine verdickte Zubereitung enthaltend ein Polymer nach einem der vorangehenden Patentansprüche. Besonders bevorzugt ist es, wenn die Einsatzkonzentration des Polymers zur Verdickung wässriger, bevorzugt tensidhaltiger wässriger Zubereitungen 2 bis 3 %, beträgt. Dadurch wird die Zubereitung bei pH-Werten <6,5 transparent und weist eine Viskosität von mindestens 3000mPa.s auf, was es ermöglicht, dass sich zugesetzte Schwebestoffe nicht absetzen, wobei die Schwebestoffe Perlen, Pigmente oder Luftblasen sein können. Besonders bevorzugt ist es, wenn die erfindungsgemäßen Zubereitungen ein Gesundheitsprodukt, Reinigungsprodukt, Haushaltsprodukt, Duschgel, Anstrichmittel, Tinten, Dispergiermittel, Antiabsetzmittel, Beton- und Zementzusatzmittel, Beschichtungen, Medizinprodukt, kosmetisches Produkt oder dermatologisches Produkt sind. Die Erfindung umfasst auch die Verwendung eines erfindungsgemäßen Polymers als Verdicker, Emulgator, Dispergiermittel oder Konsistenzgeber.

Vorteilhaft werden die Zubereitungen - sofern es sich um gelformige Zubereitungen mit Fließgrenze handelt - so ausgestaltet, dass sie eine Fließgrenze von 0,5-20 Pa aufweisen, bevorzugt 1-6 Pa.

Als Fließgrenze wird die kritische Schubspannung der Fließkurve angesehen. Sie kann erfindungsgemäß wie folgt ermittelt werden:
Gemessen wird die Fließkurve auf einem schubspannungsgesteuerten Rheometer bei 25°C ± 1°C mit 20 mm Platte/Platte Geometrie bei einem Spalt zwischen 0,8 mm und 1,2 mm, wobei strukturschonend befüllt wird. Es wird eine geeignete konstante Schubspannungszeitrampe vorgegeben und vor dem Test eine entsprechende Strukturerholungszeit eingehalten und die kritische Schubspannung im Maximum der Fließkurve angegeben.

Vorteilhaft werden die Zubereitungen so ausgestaltet, dass sie einen tan δ von 0,05 0,6 aufweisen, bevorzugt 0,1 - 0,5.

Unter tan δ wird erfindungsgemäß der Quotient aus dem Verlustmodul und dem Speichermodul verstanden. Der tan δ wird wie folgt ermittelt:
Gemessen werden Verlust- und Speichermodul durch einen dynamischen Frequenztestlauf einem schubspannungsgesteuerten Rheometer bei 40°C ± 1 °C mit 20 mm Platte/Platte Geometrie bei einem Spalt zwischen 0,8 mm und 1,2 mm, wobei strukturschonend befüllt wird. Es wird nach dem Stand der Technik der Frequenztest mit einer entsprechenden Strukturerholungszeit vor dem Test durchgeführt und der tan δ im Frequenzbereich zwischen 0,05 rad/s und 3,0 rad/s angegeben, bevorzugt zwischen 0,08 rad/s und 1,0 rad/s.

Vorteilhaft werden die Zubereitungen so ausgestaltet, dass Sie bei pH < 6.2 einen Trübungswert von NTU (Nephelometric Turbidity Unit) < 20 aufweisen. Der Trübungswert wird gemessen mit einem Trübungsmessgerät, wobei destilliertes Wasser mit einem Wert von NTU=0 als Standard gilt.

Erfindungsgemäß ist es auch, wenn nicht Wasser, sondern andere polare Flüssigkeiten durch erfindungsgemäße Polymere verdickt werden. Insbesondere können Alkohole, Glykole, Polyole, Amine und organische Säuren wie etwa Acrylsäure verdickt werden.

Erfindungsgemäß ist auch die Verwendung erfindungsgemäßer Polymere als Emulgator. So können Cremes und Lotionen bereitgestellt werden.

Die Polymere eignen sich auch als Dispergier- und Antiabsetzmittel.

### Beispiele

### Synthese der Copolymere

Die nachfolgenden Beispiele sollen die vorliegende Erfindung verdeutlichen, ohne sie einzuschränken. Die Herstellung der Beispiele erfolgt gemäß der nachstehend beschriebenen Methode, wobei Art und Mengen der jeweils als Ausgangskomponenten eingesetzten Monomere, Kettenübertragungsmittel und Schutzkolloide in der Tabelle 2 zusammengefasst wiedergegeben sind. Teile und Prozente beziehen sich auf das Gewicht.

### Beispiel 1:

| | |
|---|---|
| Monomerphase | |
| Methacrylsäure | 37,000 Teile |
| Ethylacrylat | 51,800 Teile |
| Octadecylacrylat | 5,000 Teile |
| Emulsogen R307 | 6,000 Teile |
| Trimethylolpropantriacrylat Wasserphase | 0,300 Teile |
| Wasser | 33,060 Teile |
| Natriumlaurylsulfat Reaktionsgefäß | 0,984 Teile |
| Wasser | 173,146 Teile |
| Natriumlaurylsulfat Initiatorphase A | 0,300 Teile |
| Wasser | 1,911 Teile |
| Ammoniumpersulfat Initiatorphase B | 0,069 Teile |
| Wasser | 2,909 Teile |
| Ammoniumpersulfat | 0,021 Teile |

Im Reaktionsgefäß, das mit Rührer, Rückflusskühler, Stickstoffzuleitung, Dosiervorrichtung und Innenthermometer ausgestattet ist, werden 173,146 Teile Wasser und 0,300 Teile Natriumlaurylsulfat vorgelegt Die Mischung wird unter Rühren und Stickstoffatmosphäre auf 82°C erwärmt.

Das Monomerengemisch wird in einem zweiten Rührgefäß hergestellt, das mit Rührer und Stickstoffzuleitung ausgestattet ist. Dazu wird die Monomerphase mit 37,000 Teilen Methacrylsäure, 51,800 Teilen Ethylacrylat, 5,000 Teilen Octadecylacrylat, 6,000 Teilen Emulsogen R307 und 0,300 Teilen Trimethylolpropantriacrylat vorlegt und In diese unter Rühren und Stickstoffatmosphäre die Wasserphase mit 33,060 Teilen Wasser und 0,984 Teilen Natriumlaurylsulfat gemischt.

Sobald im Reaktionsgefäß eine Temperatur von 82°C erreicht ist, wird eine Initiatorphase A, bestehend aus 0,069 Teilen Ammoniumpersulfat und 1,911 Teilen Wasser zugegeben und das Monomerengemisch bei 85-88°C während 30 Minuten gleichmäßig zudosiert. Danach wird eine Initiatorphase B, bestehend aus 0,021 Teilen Ammoniumpersulfat und 2,909 Teilen Wasser zugegeben und anschließend die Reaktionsmischung noch 4 Stunden bei 90°C nachpolymerisiert, bevor auf <40°C abgekühlt wird.

### Beispiele 2- 51:

Die nachfolgenden Beispiele (Tabelle 1) sind analog zu Beispiel 1 hergestellt. In den meisten Beispielen wurde Natriumlaurylsulfat als Emulgator eingesetzt. Abweichend wurden die Beispiele 5 und 11 aus einer Kombination von Natriumlaurylsulfat und ethoxyliertem Isotridecylalkohol (Genapol X1005, Clariant) und Beispiel 10 mit Isotridecylalkohol (Genapol X1005, Clariant) hergestellt.

### Abkürzungen

- MAA: Methacrylsäure
- EA: Ethylacrylat
- ODA: Octadecylacrylat
- LA: Laurylacrylat
- S20W: Bisomer® S20W (Cognis) Methoxypolyethyleneglykolmethacrylat (EO 45 mol)
- R307: Emulsogen® R307 (Clariant) EO/PO 30 1,4-Butandiolvinylether (EO/PO 30 mol)
- RAL307: Emulsogen® RAL307 (Clariant) Allylpolyalkylenglykolether (EO 30 mol)
- R208: Emulsogen® R208 (Clariant) 1,4-Butandiolvinylether (EO/PO 25 mol)
- A11/1800: Polyglycol A11/1800 (Clariant) Allylpolyalkylenglykolether (EO 20 mol, PO 20 mol)
- R500: Polyglycol R500 (Clariant) Vinylpolyalkylenglykolether (EO 9 mol)
- R1100: Polyglycol R1100 (Clariant) Vinylpolyalkylenglykolether (EO 20 mol)
- R2000: Polyglycol R2000 (Clarlant) Vinylpolyalkylenglykolether (EO 40 mol)
- AM 20/20: Polyglycol AM 20/20 (Clariant) Polyalkylenglykolallylmethylether (EO 20 mol, PO 20 mol)
- AB 1500: Polyglycol AB1500 (Clariant) Polypropylenglykolallylbutylether (PO 25 mol)
- AB/25-8: Polyglycol AB/25-8 (Glariant) Polyalkylenglykolallylbutylether (EO 25 mol, PO 8 mol)
- A31/1600: Polyglycol A31/1600 (Clariant) Polyalkylenglykolallylether (EO 25 mol, PO 8 mol)
- A 10 R: Pluriol® A10R (BASF) Allylalkoholalkoxylate
- A 111 R: Pluriol® A111R (BASF) Allylalkoholalkoxylate
- SPE: RALU®MER SPE (Raschig) Dimethyl[2-[(2-methyl-1-oxoallyl)oxy]ethyl](3-sultopropyl)ammoniumhydroxid
- SPM: RALU®MER SPM (Raschig) Kallum-3-sulfopropylmethacrylat
- SPP: RALU®MER SPP (Raschig) Dimethyl[3-[(2-methyl-1-oxoallyl)amino]propyl]-3-sulfopropylamrnoniurnhydroxid
- TMPTA: Trimethylolpropantriacrylat
- TMPTMA: Trimethylolpropantrlmethacrylat
- IMP: Isooctyl-3-mercaptopropianat
- AMHEC: Tylose AM H40 YP2 (SE Tylose) Allyl-modifizierte Hydroxyethylcellulose

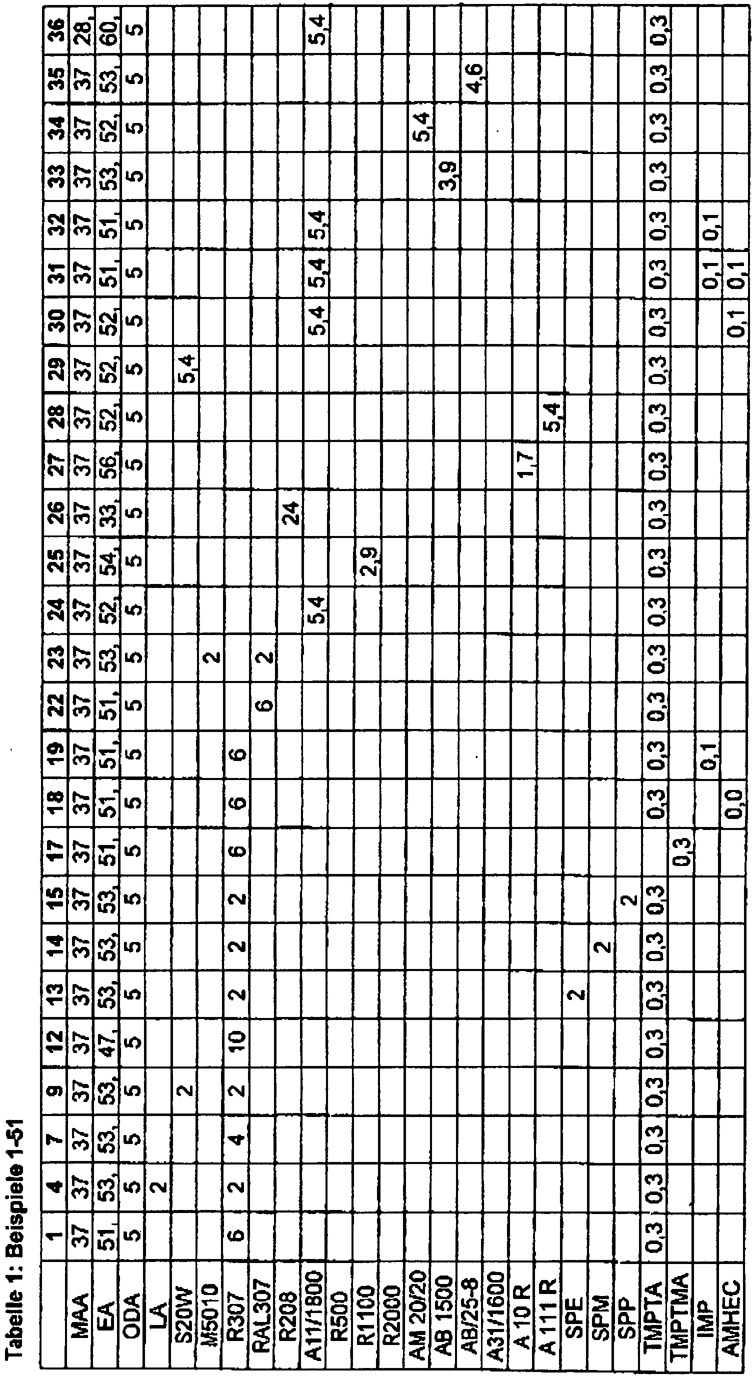

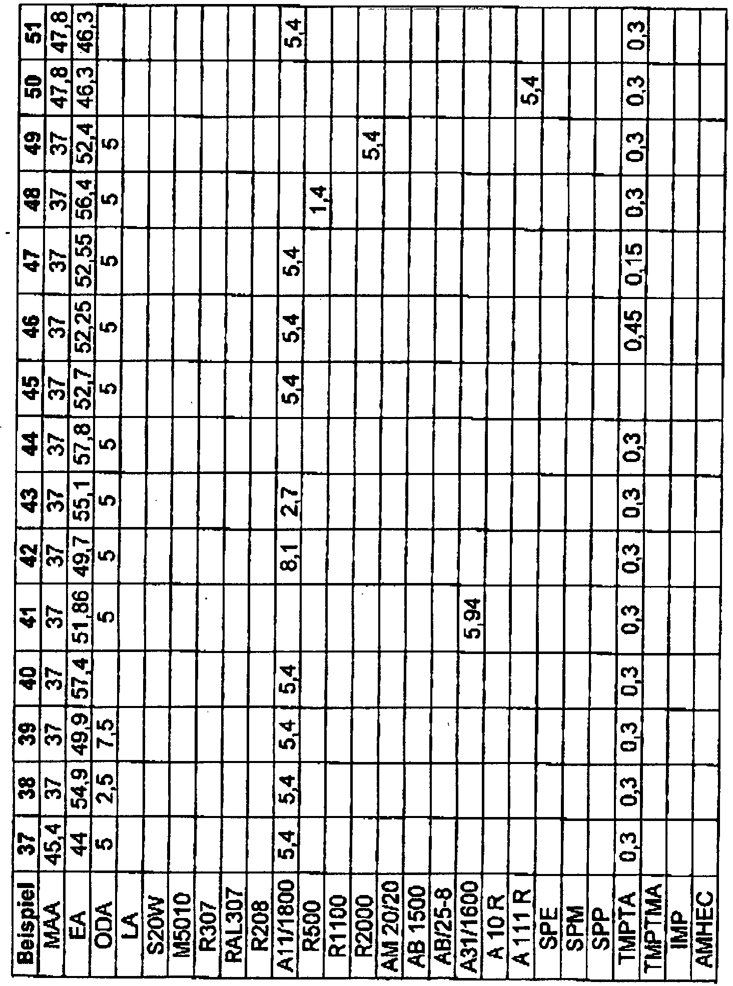

**Tabelle 2: Ergebnisse Polymeremulsionen**

| Beispiel | pH-Wert | Partikelgröße* in nm |
|---|---|---|
| 1 | 2,48 | 108 |
| 4 | n.b. | n.b. |
| 7 | n.b. | n.b. |
| 9 | n.b. | n.b. |
| 12 | n.b. | n.b. |
| 13 | 2,57 | 102 |
| 14 | 2,62 | 129 |
| 15 | 3,13 | 95 |
| 17 | 2,53 | 88 |
| 18 | 2,52 | 107 |
| 19 | 2,51 | 101 |
| 22 | 2,50 | 96 |
| 23 | 2,52 | 89 |
| 24 | 2,55 | 87 |
| 25 | 2,47 | 111 |
| 26 | 2,67 | 165 |
| 27 | 2,76 | 89 |
| 28 | 2.50 | 87 |
| 29 | 2,56 | 103 |
| 30 | 2,56 | 87 |
| 31 | 2,57 | 95 |
| 32 | 2,48 | 79 |
| 33 | 2,30 | 104 |
| 34 | 2,37 | 96 |
| 35 | 2,46 | 85 |
| 36 | 2,36 | 85 |
| 37 | 2,46 | 113 |
| 38 | 2,39 | 89 |
| 39 | 2,37 | 96 |
| 40 | 2,38 | 94 |
| 41 | 2,65 | 85 |
| 42 | 2,32 | 103 |
| 43 | 2,42 | 91 |
| 44 | 2,39 | 90 |
| 45 | 2,36 | 87 |
| 46 | 2,42 | 101 |
| 47 | 2,34 | 84 |
| 48 | 2,40 | 106 |
| 49 | 2,40 | 123 |
| 50 | 2,69 | 100 |
| 51 | 2,46 | 99 |

| | | |
|---|---|---|
| *Die Partikelgröße in der Polymeremulsion wird mit Dynamischer Lichtstreuung (DLS) in einer verdünnten wässrigen Probe der Emulsion bei 25°C ± 1 °C ermittelt. | | |

### Beispiel 52:

Die nachfolgende Zubereitung soll die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitung bezogen.

Das erfindungsgemäße Polymer wird mit einem Teil der Wasserphase verdünnt und unter Rühren zur Tensidphase gegeben. Anschließend werden die weiteren Rezepturbestandteile bis auf NaOH und die Schwebekörper unter Rühren zugegeben. Nach erfolgter pH-Einstellung werden die Schwebekörper in die fertige Gelgrundlage unter möglichst geringer Scherung eingerührt.

**Tabelle 3: Zubereitung Duschgel**

| | |
|---|---|
| Natrium Laurethsulfat | 6,50 |
| Cocoamidopropylbetain | 4,60 |
| Natriumcocoylglutamat | 0,50 |
| Polymer | 2,25 |
| PEG-40 hydriertes Rizinusöl | 0,80 |
| PEG-7 Glyceryl Cocoat | 1,75 |
| Pigmente | q.s. |
| NaOH | q.s. |
| Parfum | q.s. |
| Wasser | ad 100 |

**Tabelle 4: Ergebnisse Zubereitung Duschgel,**

| Beispiel | tan δ | Viskosität in mPas | pH-Wert Duschgel |
|---|---|---|---|
| 1 | 0,30 | 3544 | 6,19 |
| 4* | 8,58 | 1977 | 6,18 |
| 7* | 1,56 | 1967 | 6,18 |
| 9* | 1,54 | 2043 | 6,18 |
| 12* | 8,14 | 1465 | 6,11 |
| 13 | 0,32 | 2404 | 6,18 |
| 14 | 4,54 | 2055 | 6,25 |
| 15 | 0,70 | 1964 | 6,18 |
| 17 | 3,57 | 3356 | 6,18 |
| 18 | 0,31 | 2092 | 6,20 |
| 19 | 1,12 | 1998 | 6,18 |
| 22 | 0,37 | 3118 | 6,23 |
| 23 | 0,35 | 3089 | 6,22 |
| 24 | 0,17 | 3409 | 6,20 |
| 25 | 0,19 | 4100 | 6,22 |
| 26 | 4,42 | 3592 | 5,86 |
| 27 | 0,15 | 2967 | 6,17 |
| 28 | 0,14 | 3301 | 6,15 |
| 29 | 2,58 | 2442 | 6,18 |
| 30 | 2,54 | 1941 | 6,21 |
| 31 | 0,52 | 2233 | 6,15 |
| 32 | 1,83 | 2752 | 6,15 |
| 33 | 0,79 | 2953 | 6,20 |
| 34 | 0,83 | 2208 | 6,21 |
| 35 | 0,24 | 3501 | 6,20 |
| 36 | 0,34 | 2833 | 6,38 |
| 37 | 0,23 | 3432 | 5,91 |
| 38 | 0,20 | 3262 | 6,18 |
| 39 | 0,26 | 3580 | 6,11 |
| 40 | 0,16 | 4120 | 6,25 |
| 41 | 0,39 | 3317 | 6,19 |
| 42 | 0,17 | 3230 | 6,15 |
| 43 | 0,25 | 3690 | 6,15 |
| 44 | 0,22 | 4167 | 6,28 |
| 45 | 5,97 | 3196 | 5,97 |
| 46 | 0,60 | 2385 | 6,14 |
| 47 | 0,26 | 4349 | 6,17 |
| 48 | 0,22 | 4073 | 6,29 |
| 49 | 0,42 | 3313 | 6,09 |
| 50 | 0,37 | 3827 | 5,87 |
| 51 | 0,83 | 3580 | 5,93 |

| | | | |
|---|---|---|---|
| *Die Beispiele wurden ohne Parfum im Duschgel hergestellt. | | | |

Die Viskosität der Zubereitungen wird auf einem Rheometer bei 25°C ± 1 °C mit 40 mm Kegel/Platte Geometrie (1° Kegelwinkel) bei einem Spalt von 0,03 mm gemessen, wobei strukturschonend befüllt wird. Es wird eine geeignete konstante Scherratenzeitrampe vorgegeben und vor dem Test eine entsprechende Strukturerholungszeit eingehalten. Die Viskosität wird bei einer Scherrate von 10 s⁻¹ angegeben.

### Beispiel 53:

Es hat sich überraschend herausgestellt, dass die durch eine schnelle Dosierung des Monomerengemisches erzeugte Morphologie der Polymere vorteilhaft in Sinne der vorliegenden Erfindung ist. Die Tabelle 5 zeigt die unterschiedlichen Eigenschaften zweier Polymere auf, wenn die Dosierzeit variiert wird. Die Dosierzeit beträgt bevorzugt 40 Minuten und besonders bevorzugt 30 Minuten.

**Tabelle 5: Dosierzeit und Ergebnisse**

| Polymer | Dosierzeit | tan δ | Viskosität in mPas |
|---|---|---|---|
| Beispiel 35 | 30 Minuten | 0,24 | 3501 |
| Beispiel 35 | 40 Minuten | 0,21 | 4114 |
| Beispiel 35 | 50 Minuten | 0,49 | 4056 |
| Beispiel 35 | 60 Minuten | 1,45 | 3585 |
| Beispiel 24 | 30 Minuten | 0,17 | 3409 |
| Beispiel 24 | 50 Minuten | 24,26 | 1805 |

### Beispiel 54:

Die erfindungsgemäßen Polymere müssen über eine ausreichende Lagerstabilität verfügen. Hierzu wurde aus den Beispielen 1, 22, 24, 25 und 28 nach einem Jahr erneut ein Duschgel nach Beispiel 52 hergestellt. Es zeigt sich, dass die erfindungsgemäßen Polymere auch nach längerer Lagerung bei Raumtemperatur vorteilhaft sind.

**Tabelle 6: Ergebnisse Lagerstabilität**

| Polymer | nach 24h | | nach Lagerung | | |
|---|---|---|---|---|---|
| | tan δ | Viskosität in mPas | Zeit | tan δ | Viskosität in mPas |
| Beispiel 1 | 0,30 | 3544 | 15 Monate | 0,24 | 4285 |
| Beispiel 22 | 0,37 | 3118 | 13 Monate | 0,29 | 4247 |
| Beispiel 24 | 0,17 | 3409 | 13 Monate | 0,20 | 4083 |
| Beispiel 25 | 0,19 | 4100 | 13 Monate | 0,22 | 4823 |
| Beispiel 28 | 0,14 | 3301 | 10 Monate | 0,20 | 3754 |

### Beispiel 55:

Im Vergleich zu anderen Polymeren beseitigen die erfindungsgemäßen Polymere im Vergleich zu anderen Polymeren Mängel des Standes der Technik.

Um dies zu verdeutlichen wurden aus den Beispielen 24, 37 und Carbopol® AQUA SF-1 (Lubrizol) jeweils ein Duschgel nach Beispiel 52 hergestellt.

**Tabelle 7: Ergebnisse im Vergleich zu AQUA SF-1**

| | Beispiel 24 | Beispiel 37 | Carbopol® AQUA SF-1 |
|---|---|---|---|
| tan δ | 0,17 | 0,23 | 0,42 |
| Viskosität in mPas | 3409 | 3432 | 3834 |
| Fließgrenze in Pa | 3,6 | 3,4 | 3,3 |
| pH-Wert | 6,20 | 5,91 | 6,45 |
| Trübung in NTU | 8 | 9 | 18 (58 bei pH 6,20). |

Die erfindungsgemäßen Polymere weisen bei niedrigerem pH-Wert als AQUA SF-1 einen besseren tan δ und eine geringere Trübung auf. Trübungswerte von <10 sind mit Quellwasser vergleichbar. Bei Einstellung einer Zubereitung mit AQUA SF-1 auf einen niedrigeren pH-Wert, z. B.: 6,20, ist die Zubereitung deutlich wahrnehmbar trüb (NTU = 58).

## Patentansprüche

1. Polymer erhältlich durch radikalische Emulsionspolymerisation von
a) wenigstens einem sauren Vnylmonomer oder dessen Salz (A),
b) wenigstens einem nichtionischen Vinylmonomer, besonders bevorzugt einem hydrophoben nichtionischen Vinylmonomer (B),
c) wenigstens einem Monomer, enthaltend eine ungesättigte Endgruppe und einen Polyoxyalkylenteil (C),
d) wenigstens einem quervernetzenden Monomeren (D),
e) optional einem Schutzkolloid (E),
**dadurch gekennzeichnet, dass** die Polymerisation so gesteuert wird, dass zumindest zeitweise der Geleffekt auftritt, dadurch erreicht, das die Monomerzugabe (Dosierzeit),während 40 Minuten besonders bevorzugt während 30 Minuten erfolgt und **dadurch gekennzeichnet, dass** (F) assoziative Monomere fehlen.

2. Polymer nach Patentanspruch1, **dadurch gekennzeichnet, dass** das saure Vinylmonomer (A) gewählt wird unter Vinylmonomeren mit Carboxylgruppen, besonders bevorzugt Acrylsäure oder Methacrylsäure oder deren Alkali-, Erdalkali-, Ammonium oder Alkylamrnonlumsalzen, ganz besonders bevorzugt Methacrylsäure oder deren Alkali-, Erdalkali-, Ammonium oder Alkylammoniumsalze.

3. Polymer nach einem der vorangehenden Patentansprüche, **dadurch gekennzeichnet, dass** das nichtionische Vinylmonomer (B) gewählt wird unter C1-C22-Alkyl-(meth)acrylaten und deren Mischungen.

4. Polymer nach einem der vorangehenden Patentansprüche, **dadurch gekennzeichnet, dass** das Monomer (C), enthaltend eine ungesättigte Endgruppe und einen Polyoxyalkylenteil gewählt wird unter Vinylpolyalkylenglykolen oder polymerisierbaren Tensiden oder deren Mischungen, besonders bevorzugt gewählt wird unter EO/PO 30 1,4-Butandiolvinylether (EO/PO 30 mol), Allylpolyalkylenglykolether (EO 30 mol), Allylpolyalkylenglykolether (EO 20 mol, PO 20 mol), Vinylpolyalkylenglykolether (EO 20 mol), Allylalkoholalkoxylat und Polyalkylenglykolallylether (ED 25 mol, PO 8 mol).

5. Polymer nach einem der vorangehenden Patentansprüche, **dadurch gekennzeichnet, dass** das quervernetzende Monomer (D) gewählt wird unter Polyol(meth)acrylaten mit wenigstens zwei (Meth)acrylatgruppen und den Mischestem von Polyolen mit Acrylsäure und/oder Methacrylsäure.

6. Polymer nach einem der vorangehenden Patentansprüche **dadurch gekennzeichnet, dass** die Monomere
a) in Gehalten von 10 bis 75 %, bevorzugt 30 bis 50 %, besonders bevorzugt 35 bis 49 %,
b) in Gehalten von 10 bis 90 %, bevorzugt 30 bis 80 %, besonders bevorzugt 47 bis 60 %,
c) in Gehalten von 0,5 bis 40 %, bevorzugt 1 bis 10 %, besonders bevorzugt 2 bis 6 %,
d) in Gehalten von bis zu 1 %, bevorzugt 0,05 bis 0,5 %, besonders bevorzugt 0,1 bis 0,35 %
vorliegen.

7. Polymer nach einem der vorangehenden Patentansprüche, **dadurch gekennzeichnet, dass** die Monomere (A):(B) in Massenverhältnissen von 1:2 bis 2:1 vorliegen.

8. Polymer nach einem der vorangehenden Patentansprüche, **dadurch gekennzeichnet, dass** es unter den Polymeren
22, erhältlich aus 37,0 Teilen Methacrylsäure, 51,0 Teilen Ethylacrylat. 5,0 Teilen Octadecylacrylat, 6,0 Teilen Allylpolyalkylenglykolether (EO 30 mol), 0,3 Teilen Trimethylolpropantriacrylat,
24, erhältlich aus 37,0 Teilen Methacrylsäure, 52,0 Teilen Ethylacrylat, 5,0 Teilen Octadecylacrylat, 5,4 Teilen Allylpolyalkylenglykolether (EO 20 mol, PO 20 mol), 0,3 Teilen Trimethylolpropantriacrylat,
25, erhältlich aus 37,0 Teilen Methacrylsäure, 54,0 Teilen Ethylacrylat, 5,0 Teilen Octadecylacrylat, 2,9 Teilen Vinylpolyalkylenglykolether (EO 20 mol), 0,3 Teilen Trimethylolpropantriacrylat,
28, erhältlich aus 37,0 Teilen Methacrylsäure, 52,0 Teilen Ethylacrylat, 5,0 Teilen Octadecylacrylat, 5,4 Teilen Allylalkoholalkoxylate, 0,3 Teilen Trimethylolpropantriacrylat,
35, erhältlich aus 37,0 Teilen Methacrylsäure, 53,1 Teilen Ethylacrylat, 5,0 Teilen Octadecylacrylat, 4,63 Teilen Polyalkylenglykolallylbutylether (EO 25 mol, PO 8 mol), 0,3 Teilen Trimethylolpropantriacrylat.
37, erhältlich aus 45,0 Teilen Methacrylsäure, 44,0 Teilen Ethylacrylat, 5.0 Teilen Octadecylacrylat, 5,4 Teilen Allylpolyalkylenglykolether (EO 20 mol, PO 20 mol), 0,3 Teilen Trimethylolpropantriacrylat, oder
40, erhältlich aus 37,0 Teilen Methacrylsäure, 57,0 Teilen Ethylacrylat, 5,4 Teilen Allylpolyalkylenglykolether (EO 20 mol, PO 20 mol), 0,3 Teilen Trimethytolpropantriacrylat,
gewählt wird.

9. Verdickte Zubereitung enthaltend ein Polymer nach einem der vorangehenden Patent-ansprüche.

10. Zubereitung nach Patentanspruch 9,**dadurch gekennzeichnet, dass** die Einsatzkonzentration des Polymers zur Verdickung wässriger, bevorzugt tensidhaltiger wässriger Zubereitungen 2 bis 3 %, beträgt.

11. Zubereitung nach Patentanspruch 9 oder 10, **dadurch gekennzeichnet, dass** sie ein Gesundheitsprodukt, Reinigungsprodukt, Haushaltsprodukt, Duschgel, Anstrichmittel, Tinten, Dispergiermittel, Antiabsetzmittel, Beton- und Zementzusatzmittel, Beschichtungen, Medizinprodukt, kosmetisches Produkt oder dermatologisches Produkt ist.

12. Verwendung eines Polymers nach einem der Patentansprüche 1 bis 9 als Verdicker, Emulgator, Dispergiermittel oder Konsistenzgeber.

## Claims

1. Polymer obtainable by free-radical emulsion polymerization of
a) at least one acidic vinyl monomer or salt thereof (A),
b) at least one nonionic vinyl monomer, particularly preferably a hydrophobic nonionic vinyl monomer (B),
c) at least one monomer containing an unsaturated end group and a polyoxyalkylene moiety (C),
d) at least one crosslinking monomer (D),
e) optionally a protective colloid (E),
**characterized in that** the polymerization is controlled such that at least at times the gel effect arises, achieved by the fact that the monomer addition (dosing time) takes place over the course of 40 minutes, particularly preferably over the course of 30 minutes, and **characterized in that** (F) associative monomers are not present.

2. Polymer according to Claim 1, **characterized in that** the acidic vinyl monomer (A) is selected from vinyl monomers with carboxyl groups, particularly preferably acrylic acid or methacrylic acid or alkali metals, alkaline earth metals, ammonium or alkylammonium salts thereof, very particularly preferably methacrylic acid or alkali metal, alkaline earth metal, ammonium or alkylammonium salts thereof.

3. Polymer according to any one of the preceding claims, **characterized in that** the nonionic vinyl monomer (B) is selected from C1-C22-alkyl (meth)acrylates and mixtures thereof.

4. Polymer according to any one of the preceding claims, **characterized in that** the monomer (C) containing an unsaturated end group and a polyoxyalkylene moiety is selected from vinylpolyalkylene glycols or polymerizable surfactants or mixtures thereof, is particularly preferably selected from EO/PO 30 1,4-butanediol vinyl ether (EO/PO 30 mol), allylpolyalkylene glycol ether (EO 30 mol), allylpolyalkylene glycol ether (EO 20 mol, PO 20 mol), vinylpolyalkylene glycol ether (EO 20 mol), allyl alcohol alkoxylate and polyalkylene glycol allyl ether (EO 25 mol, PO 8 mol).

5. Polymer according to any one of the preceding claims, **characterized in that** the crosslinking monomer (D) is selected from polyol (meth)acrylates with at least two (meth)acrylate groups and the mixed esters of polyols with acrylic acid and/or methacrylic acid.

6. Polymer according to any of one of the preceding claims, **characterized in that** the monomers
a) are present in contents of from 10 to 75%, preferably 30 to 50%, particularly preferably 35 to 49%,
b) are present in contents of from 10 to 90%, preferably 30 to 80%, particularly preferably 47 to 60%,
c) are present in contents of from 0.5 to 40%, preferably 1 to 10%, particularly preferably 2 to 6%,
d) are present in contents of up to 1%, preferably 0.05 to 0.5%, particularly preferably 0.1 to 0.35%.

7. Polymer according to any one of the preceding claims, **characterized in that** the monomers (A):(B) are present in mass ratios of from 1:2 to 2:1.

8. Polymer according to any one of the preceding claims, **characterized in that** it is selected from the polymers
22, obtainable from 37.0 parts of methacrylic acid, 51.0 parts of ethyl acrylate, 5.0 parts of octadecyl acrylate, 6.0 parts of allylpolyalkylene glycol ether (EO 30 mol), 0.3 parts of trimethylolpropane triacrylate,
24, obtainable from 37.0 parts of methacrylic acid, 52.0 parts of ethyl acrylate, 5.0 parts of octadecyl acrylate, 5.4 parts of allylpolyalkylene glycol ether (EO 20 mol, PO 20 mol), 0.3 parts of trimethylolpropane triacrylate,
25, obtainable from 37.0 parts of methacrylic acid, 54.0 parts of ethyl acrylate, 5.0 parts of octadecyl acrylate, 2.9 parts of vinylpolyalkylene glycol ether (EO 20 mol), 0.3 parts of trimethylolpropane triacrylate,
28, obtainable from 37.0 parts of methacrylic acid, 52.0 parts of ethyl acrylate, 5.0 parts of octadecyl acrylate, 5.4 parts of allyl alcohol alkoxylates, 0.3 parts of trimethylolpropane triacrylate,
35, obtainable from 37.0 parts of methacrylic acid, 53.1 parts of ethyl acrylate, 5.0 parts of octadecyl acrylate, 4.63 parts of polyalkylene glycol allyl butyl ether (EO 25 mol, PO 8 mol), 0.3 parts of trimethylolpropane triacrylate,
37, obtainable from 45.0 parts of methacrylic acid, 44.0 parts of ethyl acrylate, 5.0 parts of octadecyl acrylate, 5.4 parts of allylpolyalkylene glycol ether (EO 20 mol, PO 20 mol), 0.3 parts of trimethylolpropane triacrylate, or
40, obtainable from 37.0 parts of methacrylic acid, 57.0 parts of ethyl acrylate, 5.4 parts of allylpolyalkylene glycol ether (EO 20 mol, PO 20 mol), 0.3 parts of trimethylolpropane triacrylate.

9. Thickened preparation comprising a polymer according to any one of the preceding claims.

10. Preparation according to Claim 9, **characterized in that** the use concentration of the polymer for thickening aqueous, preferably surfactant-containing aqueous, preparations is 2 to 3%.

11. Preparation according to Claim 9 or 10, **characterized in that** it is a health product, cleaning product, household product, showergel, paint, inks, dispersant, antisettling agent, concrete and cement additive, coatings, medicinal product, cosmetic product or dermatological product.

12. Use of a polymer according to any one of Claims 1 to 9 as thickener, emulsifier, dispersant or consistency regulator.

## Revendications

1. Polymère pouvant être obtenu par polymérisation radicalaire en émulsion
a) d'au moins un monomère vinylique acide ou sel de celui-ci (A),
b) d'au moins un monomère vinylique non ionique, de façon particulièrement préférée d'un monomère vinylique non ionique hydrophobe (B),
c) d'au moins un monomère contenant un groupe terminal insaturé et un fragment polyoxyalkylène (C),
d) d'au moins un monomère réticulant (D),
e) en option d'un colloïde protecteur (E),
**caractérisé en ce qu'**on conduit la polymérisation de manière qu'apparaisse au moins temporairement l'effet de gel, réalisé par le fait que l'addition des monomères (temps d'addition dosée) s'effectue pendant 40 minutes, de façon particulièrement préférée pendant 30 minutes et **caractérisé en ce que** des monomères associatifs (F) sont absents.

2. Polymère selon la revendication 1, **caractérisé en ce que** le monomère vinylique (A) est choisi parmi des monomères vinyliques comportant des groupes carboxy, de façon particulièrement préférée l'acide acrylique ou l'acide méthacrylique ou leurs sels de métaux alcalins, de métaux alcalino-terreux, d'ammonium ou d'alkylammonium, de façon tout particulièrement préférée l'acide méthacrylique ou ses sels de métaux alcalins, de métaux alcalino-terreux, d'ammonium ou d'alkylammonium.

3. Polymère selon l'une quelconque des revendications précédentes, **caractérisé en ce que** le monomère vinylique non ionique (B) est choisi parmi des (méth)acrylates d'alkyle en C₁-C₂₂ et des mélanges de ceux-ci.

4. Polymère selon l'une quelconque des revendications précédentes, **caractérisé en ce que** le monomère (C), contenant un groupe terminal insaturé et un fragment polyoxyalkylène, est choisi parmi des vinylpolyalkylèneglycols ou des tensioactifs polymérisables ou des mélanges de ceux-ci, de façon particulièrement préférée est choisi parmi le 1,4-butanediolvinyléther EO/PO 30 (30 moles d'EO/PO), des allylpolyalkylèneglycoléthers (30 moles d'EO), allylpolyalkylèneglycoléthers (20 moles d'EO, 20 moles de PO), vinylpolyalkylèneglycoléthers (20 moles d'EO), un produit d'alcoxylation d'alcool allylique et un éther allylique de polyalkylèneglycol (25 moles d'EO, 8 moles de PO).

5. Polymère selon l'une quelconque des revendications précédentes, **caractérisé en ce que** le monomère réticulant (D) est choisi parmi des (méth)acrylates de polyols comportant au moins deux groupes (méth)acrylate et les esters mixtes de polyols avec l'acide acrylique et/ou l'acide méthacrylique.

6. Polymère selon l'une quelconque des revendications précédentes, **caractérisé en ce que** les monomères sont présents
a) en des proportions de 10 à 75 %, de préférence 30 à 50 %, de façon particulièrement préférée 35 à 49 %,
b) en des proportions de 10 à 90 %, de préférence 30 à 80 %, de façon particulièrement préférée 47 à 60 %,
c) en des proportions de 0,5 à 40 %, de préférence 1 à 10 %, de façon particulièrement préférée 2 à 6 %,
d) en des proportions de jusqu'à 1 %, de préférence 0,05 à 0,5 %, de façon particulièrement préférée 0,1 à 0,35 %.

7. Polymère selon l'une quelconque des revendications précédentes, **caractérisé en ce que** les monomères (A):(B) sont présents en des rapports massiques de 1:2 à 2:1.

8. Polymère selon l'une quelconque des revendications précédentes, **caractérisé en ce que** parmi les polymères on choisit
22, pouvant être obtenu à partir de 37,0 parties d'acide méthacrylique, 51,0 parties d'acrylate d'éthyle, 5,0 parties d'acrylate d'octadécyle, 6,0 parties d'allylpolyalkylèneglycoléther (30 moles d'EO), 0,3 partie de triacrylate de triméthylolpropane,
24, pouvant être obtenu à partir de 37,0 parties d'acide méthacrylique, 52,0 parties d'acrylate d'éthyle, 5,0 parties d'acrylate d'octadécyle, 5,4 parties d'allylpolyalkylèneglycoléther (20 moles d'EO, 20 moles de PO), 0,3 partie de triacrylate de triméthylolpropane,
25, pouvant être obtenu à partir de 37,0 parties d'acide méthacrylique, 54,0 parties d'acrylate d'éthyle, 5,0 parties d'acrylate d'octadécyle, 2,9 parties de vinylpolyalkylèneglycoléther (20 moles d'EO), 0,3 partie de triacrylate de triméthylolpropane,
28, pouvant être obtenu à partir de 37,0 parties d'acide méthacrylique, 52,0 parties d'acrylate d'éthyle, 5,0 parties d'acrylate d'octadécyle, 5,4 parties de produits d'alcoxylation d'alcool allylique, 0,3 partie de triacrylate de triméthylolpropane,
35, pouvant être obtenu à partir de 37,0 parties d'acide méthacrylique, 53,1 parties d'acrylate d'éthyle, 5,0 parties d'acrylate d'octadécyle, 4,63 parties de polyalkylèneglycolallylbutyléther (25 moles d'EO, 8 moles de PO), 0,3 partie de triacrylate de triméthylolpropane,
37. pouvant être obtenu à partir de 45,0 parties d'acide méthacrylique, 44,0 parties d'acrylate d'éthyle, 5,0 parties d'acrylate d'octadécyle, 5,4 parties d'allylpolyalkylèneglycoléther (20 moles d'EO, 20 moles de PO), 0,3 partie de triacrylate de triméthylolpropane, ou 40, pouvant être obtenu à partir de 37,0 parties d'acide méthacrylique, 57,0 parties d'acrylate d'éthyle, 5,4 parties d'allylpolyalkylèneglycoléther (20 moles d'EO, 20 moles de PO),
0,3 partie de triacrylate de triméthylolpropane,

9. Préparation épaississante contenant un polymère selon l'une quelconque des revendications précédentes.

10. Préparation selon la revendication 9, **caractérisée en ce que** la concentration utilisée du polymère pour l'épaississement de préparations aqueuses contenant de préférence des tensioactifs vaut de 2 à 3 %.

11. Préparation selon la revendication 9 ou 10, **caractérisée en ce qu'**il s'agit d'un produit d'hygiène, produit de nettoyage, produit pour la maison, gel douche, d'une peinture, d'encres, d'un dispersant, d'un agent anti-dépôt, d'additifs pour ciment ou béton, de revêtements, d'un produit médical, produit cosmétique ou produit dermatologique.

12. Utilisation d'un polymère selon l'une quelconque des revendications 1 à 9 en tant qu'épaississant, dispersant ou agent de consistance.
